Europäisches Patentamt

**European Patent Office**

**Office européen des brevets**

(11) Publication number: **0 153 875**
**A2**

# EUROPEAN PATENT APPLICATION

(21) Application number: 85301403.3

(22) Date of filing: 28.02.85

(51) Int. Cl.⁴: **G 01 N 33/543**

(30) Priority: 01.03.84 AU 3870/84

(43) Date of publication of application:
04.09.85 Bulletin 85/36

(84) Designated Contracting States:
AT BE CH DE FR GB IT LI LU NL SE

(71) Applicant: THE STATE OF VICTORIA
Treasury Place
Victoria 3000(AU)

(72) Inventor: Spencer, Terence Leslie
30 Garden Street
Benalla Victoria(AU)

(74) Representative: Marsh, Roy David et al,
Brewer & Son 5-9, Quality Court Chancery Lane
London WC2A 1HT(GB)

(54) Enzyme-linked immunosorbent assay method and test kit.

(57) In ELISA assaying, by submitting a sample to an enzymelinked immunosorbent assay, difficulties of non-specific binding and edge effects are alleviated by adding an effective amount of a sugar and/or an alcohol or mixtures thereof. An assay method and test kit are described and claimed.

EP 0 153 875 A2

ENZYME-LINKED IMMUNOSORBENT ASSAY METHOD AND TEST KIT.

The present invention relates to an improved enzyme-linked immunosorbent assay process.

The enzyme-linked immunosorbent assay (ELISA) has become a significant addition to existing serological tools over the last few years. Although the process is easy to perform and quite sensitive, there are certain problems to be solved in relation to its use. In particular, two problems related to the prior art which have caused considerable difficulties are "non-specific" binding and "edge effects".

In relation to non-specific binding, readings obtained from the ELISA process are limited in their sensitivity and accuracy as they are often substantially higher than normal. This is so as the binding of the conjugate is often less discriminatory than is desirable. In other words, binding occurs other than with the specific antibodies or antigens the ELISA processes are designed to identify. This is known as "non-specific" binding. Moreover, a quantitive estimation of the amount of such specific antibodies or antigens is made more difficulty by non-specific binding.

Further, in certain enzyme-linked immunosorbent assays adsorption of the antigen or antibody occurs on microtitre plates, usually polystyrene microtitre plates. It has been noted that with these microtitre plates, however, the readings obtained which were inconsistent and difficult to read particularly in the outer wells of the microtitre plates. This "edge effect" results in higher readings than those observed in the inner wells. Accordingly, a significant percentage of the wells on each microtitre plates are rendered substantially useless for the ELISA processes.

Whilst it is known in the prior art to utilise 1% bovine albumin to increase the specificity of such tests, this has been found to provide substantially no improvement in relation to the edge effect described above.

Accordingly, it is an object of the present invention to overcome, or at least alleviate, some of the difficulties related to the prior art.

According to the present invention there is provided a method of assaying a sample which comprises submitting the sample to an enzyme linked immunosorbent assay wherein the assay method further includes adding an effective amount of a sugar and/or an alcohol or mixtures thereof. Preferably the sugar and/or alcohol is added in an amount effective to reduce non-specific binding and/or edge effects when microtitre plates are used. Preferably the invention provides a method of assaying a sample which comprises

(a) providing a suitable vessel,

(b) coating the vessel with a suitable antigen or anti-body,

(c) adding the sample to the coated vessel,

(d) adding a conjugate to the coated vessel, and

(e) adding an effective amount of sugar and/or an alcohol or mixtures thereof.

The sample to be assayed may be of any type which is suitable for use with the ELISA method including those containing antigens or antibodies. For example, the sample may be a serological sample. Alternatively, the sample may be in the form of a plasma. A serological sample may include blood serums and the like.

The vessel useful in relation to step (a) of the method described above may be of any suitable type. Standard test tubes or microtitre plates may be utilised. The vessel may be selected to allow for absorption of the antigens or antibodies which may be used in the ELISA test. Polystyrene and similar surfaces have been found to be suitable. For example, the vessel may take the form of a polystyrene tube or microtitre plate.

Step (b) according to the present invention provides for coating the vessel with a suitable antigen or antibodies. Satisfactory antigens or antibodies can be found by performance testing against reference antibodies or antigens respectively. For example, anti-species globulin serum may be used. The immunoglobulin fraction may be used. For illustrative purposes the present invention will later be described with reference to a gamma globulin fraction of a sheep anti-rabbit IgG. The antigen may be a diluted normal animal serum pool. For example a dilution of 1:4000 may be used.

The coating step may be achieved by

(1) treating the vessel with a soluble antigen in buffer solution, and

(2) washing the thus coated vessel, preferably in a saline solution.

The buffer solution may be a carbonate buffer solution at a pH of approximately 9.4 to 9.7, preferably 9.5. The saline solution may contain a surfactant such as polysorbate. A particularly preferred polysorbate is "Tween 20".

Step (c) of the assay method provides for adding a sample to the coated vessel. The sample addition step (c) may further incubating the coated vessel for a period sufficient to allow the specific antibody within the sample to become attached to the layer of antigen. This step may be followed by a further step which comprises emptying the vessel and washing same with a saline solution. As discussed above the saline solution may include a surfactant such as a polysorbate.

Step (d) of the method according to the present provides for adding a conjugate to the coated vessel. The conjugate may be an enzyme-linked antiglobulin reagent.

The conjugate will vary as to the

(1) type of immunoglobulin preparation used, e.g. IgG (H+L), IgG (H),

(2) the animal species used for immunization e.g. sheep, rabbit.
The enzyme may be selected from any of those suitable for use in the ELISA method. For example, the enzyme may be an alkaline phosphatase, a glucose oxidase or a horseradish peroxidase. For illustrative purposes, the conjugate may be a sheep anti-rabbit IgG-horseradish peroxidase conjugate. Such a conjugate may be prepared by the method of Wilson and Nakane.

The conjugate addition step (d) may further comprise incubating the coated vessel for a period sufficient to allow reaction between the conjugate and antibody.

In a preferred form, the antigen antibody reaction and conjugate steps (c) and (d) may be conducted at low temperatures. It has been found that the edge effects and non-specific binding may be reduced if the incubation temperatures are kept low. Temperatures of approximately 4°C to approximately room temperature may be used. More preferably the incubations may be conducted at approximately room temperature.

Step (e) of the method according to the present invention provides for adding an effective amount of a sugar and/or alcohol or mixtures thereof. The addition of the sugar and/or alcohol may occur at the conjugate or antigen steps (b) or (d). Preferably the sugar and/or alcohol additive is present in an approximately 1:1 volume ratio to the antigen or conjugate. Desirably, the addition occurs at the conjugate step.

The sugar, when present, may be of any suitable type e.g. the sugar may be selected from sucrose, glucose, mannose, lactose, maltose, raffinose, ionsitol, galactose and rhamnose. Sucrose is preferred.

Sugars may be present in concentrations of approximately 0 to 20% by weight. Preferably the sugars are present in concentrations of approximately 2 to 10% preferably 5 to 7% by weight.

The alcohols, when present, may be of any suitable type, e.g. the alcohols may be selected from polyhydric alcohols. Glycerol is preferred. The alcohols may be present in concentration of from 0 to 20% by weight. Preferably 2 to 10% by weight, more preferably 5 to 10% by weight is used.

Particularly, effective results in reduction of edge effects and/or non-specific binding are achieved with a 10% sucrose/10% glycerol composition.

In a still further aspect of the present invention there is provided a test kit for assaying a sample which test kit includes

(a) a vessel suitable for use in an enzyme-linked immunosorbent assay,

(b) an enzyme conjugate in a suitable container,

(c) an antigen in a suitable container,

(d) a sugar and/or an alcohol or mixtures thereof in a suitable container or containers.

Preferably, the component (d) comprises a 10% sucrose/10% glycerol solution in a suitable container. More preferably, the vessel (a) is a microtitre plate.

The present invention will now be more fully described with reference to the accompanying example. It should be understood that this example is illustrative only and should not be taken in any way as a restriction on the generality of the invention described above.

EXAMPLE 1

Experiments described in the accompanying tables 1 to 6 were conducted on microtitre plates as follows:

| Plate Type: | 'U' Bottom Cooke M24A |
|---|---|
| Reagent Volumes: | 50ul (Except Substrate - 100ul) |
| Coating Conditions: | γ Gobulin Fraction of Sheep Anti-Rabbit IgG at 10ug/ml in 0.1M $Na_2 CO_3$ pH 9.5, 0/N, RT |
| Wash/Diluent Buffer: | PBS (Phosphate-buffered saline) containing 0.05% Tween 20. |
| Antigen: | Normal Rabbit Serum Pool at 1:4000 for 2 hours at RT |
| Conjugate: | Sheep Anti-Rabbit IgG-HRPO Conjugate Prepared by the Method of Wilson and Nakand at 1:6000 for 2 hours at RT |
| Substrate: | 1mM ABTs 2.5mM $H_2O_2$ in 0.1m Citrate pH 4.2. $OD_{414}$ (Optical Density) read after 1 Hour at RT using Multiscan MC Platereader. |
| Calculations: | All $OD_{414}$ (Optical Density) Readings were Multiplied by 100 and results are then expressed as means ±SD, (Standard Deviation). 'P' Values were determined using the Method of Student. |

The exception was Table 3 where the conjugate was directly coated onto the plate and then the plate processed as above.

TABLE 1

| TEMPERATURE | 10% SUCROSE/ 10% GLYCEROL | + ANTIGEN | | | |
|---|---|---|---|---|---|
| | | OUTER | INNER | OUTER | INNER |
| 4° | − | 245 ± 13 | 249 ± 14** | 9 ± 4 | 10 ± 3** |
| | + | 141 + 9 | 155 ± 10* | 2 ± 2 | 2 ± 1** |
| RT | − | 273 ± 11 | 255 ± 10* | 26 ± 9 | 10 ± 4* |
| | + | 174 ± 10 | 171 ± 8** | 2 ± 1 | 2 ± 1** |
| 37°C | − | 303 ± 8 | 267 ± 10* | 47 ± 16 | 18 ± 6* |
| | + | 170 ± 9 | 171 ± 9** | 5 ± 2 | 3 ± 1* |

LEVELS OF SIGNIFICANCE * P < 0.001

** NS

TABLE 2

| ADDITIONS | + ANTIGEN | | − ANTIGEN | |
|---|---|---|---|---|
| | OUTER | INNER | OUTER | INNER |
| NIL | 247 ± 13 | 229 ± 9* | 8 ± 4 | 5 ± 1* |
| 5% SUCROSE | 219 ± 9 | 209 ± 9** | 3 ± 2 | 2 ± 1** |
| 10% SUCROSE | 214 ± 7 | 210 ± 6 | 3 ± 1 | 3 ± 1 |
| 20% SUCROSE | 132 ± 11 | 131 ± 10 | 2 ± 1 | 2 ± 1 |
| 5% GLYCEROL | 230 ± 13 | 220 ± 8** | 5 ± 3 | 3 ± 1** |
| 10% GLYCEROL | 210 ± 9 | 205 ± 6*** | 3 ± 1 | 2 ± 1*** |
| 20% GLYCEROL | 151 ± 8 | 151 ± 8 | 2 ± 1 | 2 ± 1 |
| 5% SUCROSE/GLYCEROL | 191 ± 9 | 188 ± 10 | 2 ± 1 | 1 ± 1*** |
| 10% SUCROSE/GLYCEROL | 168 ± 8 | 165 ± 7 | 1 ± 1 | 1 ± 1 |
| 20% SUCROSE/GLYCEROL | 70 ± 6 | 69 ± 7 | 1 ± 1 | 1 ± 1 |

LEVELS OF SIGNIFICANCE  *P < 0.001

**0.001 < P < 0.01

***0.02  < P < 0.05

TABLE 3

| COATING | CONTROL | | + 10% SUCROSE/10% GLYCEROL | |
|---|---|---|---|---|
| | OUTER | INNER | OUTER | INNER |
| 1:10,000 | 231 ± 12 | 217 ± 7* | 297 ± 11 | 280 ± 9* |
| 1:20,000 | 121 ± 5 | 117 ± 5** | 189 ± 14 | 176 ± 8* |
| 1:40,000 | 47 ± 2 | 47 ± 2 | 69 ± 4 | 67 ± 4 |

LEVELS OF SIGNIFICANCE   *P < 0.001

**0.001 < P < 0.01

## TABLE 4

| SUCROSE/ GLYCEROL (%) | + ANTIGEN | | − ANTIGEN | |
|---|---|---|---|---|
| | OUTER | INNER | OUTER | INNER |
| 0 | 226 ± 11 | 209 ± 7* | 10 ± 4 | 5 ± 1 |
| 5 | 221 ± 6 | 206 ± 5* | 7 ± 3 | 4 ± 1 |
| 10 | 196 ± 9 | 195 ± 7** | 5 ± 1 | 3 ± 1 |
| 20 | 190 ± 12 | 188 ± 6** | 6 ± 3 | 4 ± 1 |

LEVELS OF SIGNIFICANCE  *P < 0.001

** NS

TABLE 5

## TABLE 5

| CONDITIONS | POSITIVE | | NEGATIVE | |
|---|---|---|---|---|
| | OUTER | INNER | OUTER | INNER |
| CONTROL | 288 ± 15 | 275 ± 12 | 8 ± 6 | 6 ± 2 |
| +10% SUCROSE | 224 ± 8 | 218 ± 9 | 4 ± 1 | 3 ± 1 |
| +10% GLUCOSE | 225 ± 5 | 218 ± 6 | 6 ± 2 | 5 ± 2 |
| +10% MANNOSE | 215 ± 6 | 206 ± 8 | 6 ± 1 | 4 ± 1 |
| +10% LACTOSE | 218 ± 8 | 212 ± 6 | 6 ± 3 | 5 ± 2 |
| +10% MALTOSE | 218 ± 7 | 212 ± 9 | 5 ± 1 | 5 ± 1 |
| +10% RAFFINOSE | 224 ± 11 | 233 ± 7 | 9 ± 2 | 8 ± 1 |
| +10% INOSITOL | 244 ± 10 | 299 ± 7 | 6 ± 1 | 5 ± 2 |
| +10% GALACTOSE | 218 ± 7 | 208 ± 7 | 10 ± 3 | 9 ± 1 |
| +10% RHAMNOSE | 161 ± 10 | 155 ± 7 | 10 ± 2 | 11 ± 4 |

## TABLE 6

| ANTIGEN DILUTION | CONJUGATE DILUTION | | |
|---|---|---|---|
| | 1:6,000 | 1:6,000 | 1:2,000 |
| | ADDITIONS AT CONJUGATE STEP | | |
| | NIL | 10% SUCROSE/10% GLYCEROL | |
| 1:16,000 | 242 ± 14 (26.9)+ | 142 ± 10 (244) | 285 ± 9 (215) |
| 1:64,000 | 228 ± 10 (25.3) | 121 ± 6 (225) | 272 ± 12 (207) |
| 1:256,000 | 165 ± 12 (18.3) | 64 ± 6 (110) | 174 ± 7 (131) |
| 1:1,024,000 | 71 ± 3 (7.9) | 25 ± 2 (43) | 47 ± 5 (35.3) |
| 1:4,096,000 | 30 ± 3 (3.3) | 9 ± 1 (15.5) | 17 ± 2 (12.8) |
| 1:16,384,000 | 11 ± 3 (1.2)** | 2 ± 1 (3.4)* | 6 ± 2 (4.5)* |
| -VE CONTROL | 8.6 ± 3.1 | 0.6 ± 0.6 | 1.3 ± 1.1 |

+     +VE/-VE RATIO

*     SIGNIFICANTLY DIFFERENT FROM -VE (P<0.001)

**     NOT SIGNIFICANTLY DIFFERENT FROM -VE (P>0.05)

Table 1 illustrates the effect of temperature and the addition of a sucrose/glycerol mixture on the edge effects and non-specific binding which are characteristics of the ELISA process. It will be noted that edge-effects and non-specific binding increase with increasing temperatures. Accordingly, these difficulties may be reduced by conducting the incubation stages of the antigen and conjugate steps at lower temperatures. It is also clear from Table 1 that the sucrose/glycerol mixture is effective throughout the range of temperatures in substantially reducing the edge-effects and non-specific binding.

A comparison of the positive/negative ratio that is the ratio of the optical density readings with and without antigen gives a guide to the accuracy of the test. The higher the value of the positive/negative ratio the more discriminating the system. It will be noted from a comparison of the values with and without the sucrose/-glycerol mixture that the positive/negative ratio is substantially improved.

Table 2 illustrates the effect of increasing the amount of sucrose and/or glycerol. Whilst Table 2 shows that both sucrose and glycerol are effective alone and together, optimal activity, minimal edge effect and minimal non-specific binding occurred in the presence of 10% sucrose/10% glycerol.

Table 3 illustrates the effect of sucrose/glycerol on coating parameters. From Table 3 it can be seen that edge effect is present and is more pronounced at higher coating concentrations. Sucrose/glycerol increases coating efficiency but does not completely abolish edge effect, when added at this step.

Table 4 illustrates the effect of sucrose/glycerol when added at the antigen step. It will be noted that the additional sucrose/glycerol substantially diminishes edge effect, when present, at the antigen step. However, sucrose/glycerol has a minor effect on non-specific binding when present at the antigen step.

Table 5 illustrates the effect of various sugars on the ELISA process. Of the sugars tested sucrose appears to be best at minimising edge effect and decreasing non-specific binding. Similar efficiencies were observed when sugars were tested in combination with glycerol (not shown).

-13- **0153875**

Table 6 illustrates the effect of sucrose/glycerol on antigen sensitivity. It will be noted that the positive/negative ratio is significantly higher when 10% sucrose/10% glycerol is present.

Table 6 also illustrates that there is a decreased sensitivity for the antigen when 10% sucrose/10% glycerol is added. However the conjugate dilution may be decreased to improve this sensitivity without substantially effecting the positive/negative ratio. The conjugate dilution may be reduced e.g. from 1 to 6,000 to 1 to 2,000.

Finally, it is to be understood that various other modifications and/or alterations may be added without departing from the spirit of the present invention as outlined herein.

CLAIMS :-

1. A method of assaying a sample which comprises submitting the sample to an enzyme linked immunosorbent assay characterised in that the assay method further includes adding an effective amount of a sugar and/or an alcohol or mixtures thereof.

2. A method according to claim 1 characterised in that the sugar and/or alcohol is added in an amount effective to reduce non-specific binding and/or edge effects when microtitre plates are used.

3. A method of assaying a sample which comprises: (a) providing a suitable vessel; (b) coating the vessel with a suitable antigen or antibody; (c) adding the sample to the coated vessel; (d) adding a conjugate to the coated vessel; and (e) adding an effective amount of a sugar and/or alcohol or mixtures thereof.

4. A method according to claim 3 characterised in that the sugar and/or alcohol is added in amounts effective to reduce non-specific binding, and/or edge effects when a microtitre plate is used as the vessel.

5. A method according to claim 4 characterised in that the sugar and/or alcohol is added at the coating step (b) or conjugate step (d).

6. A method according to claim 5 characterised in that the sugar and/or alcohol additive is present in an approximately 1:1 volume ratio to the antigen or conjugate.

7. A method according to claim 6 characterised in that the sugar and/or alcohol is added at the conjugate step (d).

8.    A method according to any one of claims 1 to 7
characterised in that the sugar is selected from sucrose,
glycose, mannose, lactose,maltose, raffinose, inositol,
galactose and rhamnose and is present in a concentration
of approximately 2 - 10% by weight.

9.    A method according to any one of claims 1 to 8
characterised in that the alcohol is selected from the
polyhydric alcohols and is present in a concentration
of from approximately 2 - 20% by weight.

10.    A method according to any one of claims 1 to 9
characterised in that the sugar and/or alcohol additive
is a composition including sucrose and glycerol.

11.    A method according to claim 10 characterised in
that the composition comprises 10% sucrose and 10%
glycerol.

12.    A method according to any one of claims 3 to 7 or
8 to 11 as dependent on claim 3, characterised in that
the coating step (b) comprises:    (i) treating the
vessel with a suitable antigen in a buffer solution;
and (ii) washing the thus coated vessel.

13.    A method according to any one of claims 3 to 7
and 12, and 8 to 11 as dependent on claim 3, characterised
in that the sample addition step (c) further comprises:
(iii) incubating the coated vessel for a period sufficient
to allow the specific antibody within the sample to
become attached to the layer of antigen.

14.    A method according to claim 13 characterised in that
the incubation is conducted at temperatures of approximately 4°C to approximately room temperature.

15.    A method according to any one of claims 3 to 7 and 12 to 14, and 8 to 11 as dependent on claim 3, characterised in that the conjugate addition step (d) further comprises: (i) incubating the coated vessel for a period sufficient to allow reaction between the conjugate and antibody.

16.    A method according to claim 14 characterised in that the incubation is conducted at temperatures of approximately 4°C to approximately room temperature.

17.    A test kit for assaying a sample which test kit includes: (a) a vessel suitable for use in an enzyme-linked immunosorbent assay; (b) an enzyme conjugate in a suitable container; (c) an antigen in a suitable container; and (d) a sugar and/or an alcohol or mixtures thereof in a suitable container or containers.

18.    A test kit according to claim 17 characterised in that the component (d) comprises a 10% sucrose/10% glycerol solution in a suitable container.

19.    A test kit according to claim 17 or 18 characterised in that the vessel (a) is a microtitre plate.